# EUROPEAN PATENT APPLICATION

(11) **EP 1 358 905 A1**
(43) Date of publication of application: **05.11.2003**
(21) Application number: 03075787.6
(22) Date of filing: 18.03.2003
(51) Int. Cl.: A61M 39/22

(54) **Device for the administration of drugs**

(30) Priority: 30.04.2002 IT MI20020921
(71) Applicant: HAEMOPHARM INDUSTRY AG, 9490 Vaduz (LI)
(72) Inventor: Valoti, Michele, 6900 Massagno (Svizzera) (IT)
(74) Representative: Mittler, Enrico

(57) **Abstract**

A device for the administration of drugs that comprises a container with pouch (1) containing said drug and a connection line (3) for the administration of the drug to a patient is described. The connection line (3) is provided with threaded means (12) for screw connection with a defluxor (5) and with a frangible plug (7), whose breaking executable from the outside of said connection line (3) at the moment of its use allows the administration of the drug to the patient. The defluxor (5) is provided with threaded means (13) couplable with said threaded means (12) of the connection line (3) by screwing.

## Description

The present invention concerns a device for the administration of liquid type drugs or pharmacological solutions.

Devices for the administration of drugs are known that comprise a solution pouch and a first hose for the connection of the pouch with a container for a drug. The latter gets mixed with the solution present in the pouch and the entire product deriving from the mixing is then transferred into it. Such devices comprise a second hose suitable to be connected with a patient for the administration of the liquid contained in the pouch.

In a known device for the administration of drugs the second hose is connected at its free end with a connector having a perforatable external membrane. An appropriate defluxor, provided with a needle on its top, is connected with the hose by means of perforation of the membrane through the introduction of the needle in the connector. The dripping of the liquid contained in the pouch in the tray of the defluxor allows the administration of the drug to the patient.

However in such device the inattention of the patient or of the operator who introduces the needle in the perforatable membrane can cause spills of the liquid contained in the pouch out of the defluxor.

In addition the introduction of the needle into the perforatable membrane can be cause of contamination of the liquid to be administered.

In view of the state of the art herein described, scope of the present invention is to provide a device for the administration of drugs that overcomes the described disadvantages.

According to the present invention such scope is attained by means of a device for the administration of drugs comprising a pouch-like container containing said drug and a connection line for the administration of the drug to a patient, characterised in that said connection line is provided with threaded means for screw connection with a defluxor and with a frangible plug, whose breaking executable from outside of said connection line at the moment of its use allows the administration of the drug to the patient.

In addition the defluxor is in turn provided with threaded means that are couplable with said threaded means of the connection line by screwing.

Owing to the present invention it is possible to provide a device for the administration of drugs that is safer and without the danger of contamination of the known devices.

The characteristics and the advantages of the present invention will become evident from the following detailed description of an embodiment thereof, that is illustrated as a non-limiting example in the enclosed drawings, in which:
Figure 1 is a front view of a device for the administration of drugs according to the present invention;
Figure 2 is a magnified section view of a part of the device in Figure 1, in particular of a frangible plug inserted in a connection line of the device in Figure 1;
Figure 3 is a section view similar to the one in Figure 2 with the frangible plug broken.

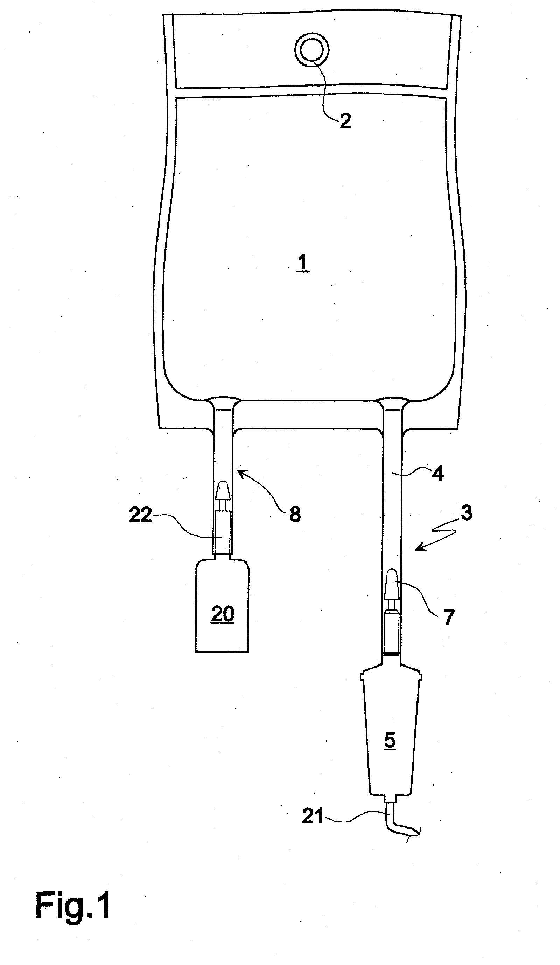

With reference to Figure 1 a device for the administration of drugs, of the liquid type or pharmacological solutions, is shown comprising a pouch container 1 with a coupling element 2 and a connection line 3 for the administration of the drug to a patient.

The connection line 3 comprises a hose 4 having a top end integral with the pouch container 1 and a bottom end couplable to a defluxor 5 provided in turn with a small hose 21 for the transport and the administration of the drug to the patient.

The hose 4 is normally kept closed by a frangible plug 7.

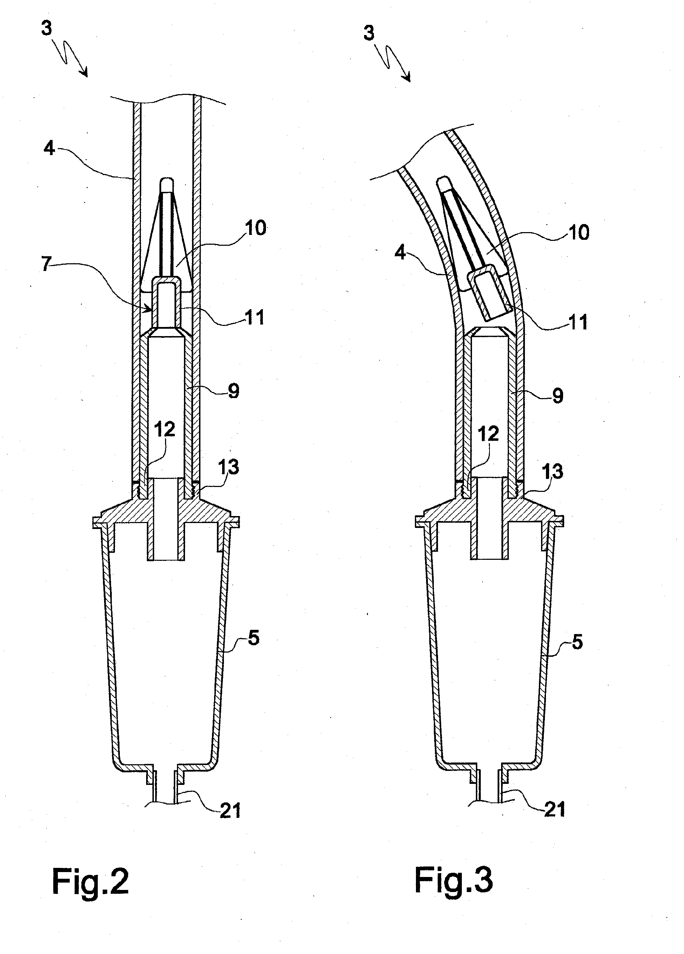

In Figures 2 and 3 a magnified section of a part of the connection line 3 is shown comprising a frangible plug 7 in two different is operating stages.

The frangible plug 7 comprises a cylindrical and hollow part 9 in which a solution can flow and an end part 11 with fins 10, that prevents such flow (Figure 2).

The cylindrical and hollow part 9 is arranged inside the hose 4 so as to be integral with it; such cylindrical and hollow part 10 comprises a final threaded part 12 that extends from the hose 4 and is suitable to engage with a threaded end 13 of the defluxor 5.

The presence of the frangible plug 7 prevents contamination of the liquid before its use, when the defluxor 5 is separated from the connection line 3.

The operation of breaking of the plug 7 is carried out at the moment of the use, that is when the patient prepares himself for the administration of the drug. More precisely, after the defluxor 5 has been attached to the line 3 by means of mutual screwing of the threaded parts 12 and 13, by applying a pressure from the outside on the connection line 3 and in particular on the part 11 of the frangible plug 7 that connects the finned end 10 with the cylindrical and hollow part 9, the breaking of the same plug is caused (figure 3) and a connection between the defluxor 5 and the pouch container 1 is created that allows the flow of the drug and its administration to the patient.

Such flow is not hindered by the finned end 10 since the same fins allow the flow of the solution into the space comprised among them.

The screwing of the defluxor 5 onto the threaded end part 12 of the frangible plug 7 creates a safe and stable coupling that prevents undesired accidental separation of the defluxor 5.

Preferably a hose 8 is provided for the connection of the pouch container 1 with a container 20 of a powdered drug that gets mixed with a solution present in the pouch so as to obtain a pharmacological solution to be administered to the patient.

In such hose 8 a frangible plug 22 as the one indicated by 7 in Figure 2 and 3 for the connection of the container of the powdered drug with the pouch container 1 can be provided.

## Claims

1. Device for the administration of drugs comprising a container with pouch (1) containing said drug and a connection line (3) for the administration of the drug to a patient, **characterised in that** said connection line (3) is provided with threaded means (12) for screw connection with a defluxor (5) and with a frangible plug (7), whose breaking executable from the outside of said connection line (3) at the moment of its use allows the administration of the drug to the patient.

2. Device according to claim 1, **characterised in that** said defluxor (5) is provided with threaded means (13) for the screw connection with said threaded means (12) of the connection line (3).

3. Device according to claim 1, **characterised in that** said frangible plug (7) comprises a cylindrical and hollow part (9) in which the drug can flow and a finned end (10) connected with said cylindrical part (9) through a frangible part (11), in such way that as a result of the breaking of said frangible part (11) said finned end (10) gets separated from said cylindrical and hollow part (9) and the drug can flow through the spaces between the fins of said finned end (10) and said cylindrical and hollow part (9).

4. Device according to claim 1, **characterised in that** said connection line (3) comprises a hose (4) connected at one end with said pouch container (1) and comprising said frangible plug (7) on its inside.

5. Device according to claim 3, **characterised in that** the cylindrical and hollow part (9) of said frangible plug (7) is arranged in integral way with the inside side surface of the hose (4).

6. Device according to claim 1, **characterised in that** said pouch container (1) provides a coupling element (2).

7. Device according to claim 1, **characterised in that** it comprises an additional connection line (8) between said pouch container (1) and an additional container for drugs (20).

8. Device according to claim 1, **characterised in that** said additional connection line (8) comprises an additional frangible plug (22).
